(19) Europäisches Patentamt
European Patent Office
Office européen des brevets

(11) Veröffentlichungsnummer: **0 286 816**
**A1**

# (12) EUROPÄISCHE PATENTANMELDUNG

(21) Anmeldenummer: **88103122.3**

(51) Int. Cl.⁴: **C07D 417/04 , A01N 43/82**

(22) Anmeldetag: **02.03.88**

(30) Priorität: **13.03.87 JP 56926/87**

(43) Veröffentlichungstag der Anmeldung: **19.10.88 Patentblatt 88/42**

(84) Benannte Vertragsstaaten: **BE CH DE ES FR GB IT LI NL**

(71) Anmelder: **NIHON TOKUSHU NOYAKU SEIZO K.K.**
**No.4, 2-Chome, Nihonbashi Honcho**
**Chuo-ku Tokyo 103(JP)**

(72) Erfinder: **Kume, Toyohiko**
**6-7-8, Asahigaoka**
**Hino-shi Tokyo(JP)**
Erfinder: **Goto, Toshio**
**3454-21, Honmachida**
**Machida-shi Tokyo(JP)**
Erfinder: **Kamochi, Atsumi**
**2-24-10, Higashi-Toyoda**
**Hino-shi Tokyo(JP)**
Erfinder: **Yanagi, Akihiko**
**2-1200-1, Oume-shi**
**Tokyo(JP)**
Erfinder: **Yagi, Shigeki**
**1-30-7, Izumi Suginami-ku**
**Tokyo(JP)**
Erfinder: **Miyauchi, Hiroshi**
**39-15, Namiki-cho**
**Hachioji-shi Tokyo(JP)**

(74) Vertreter: **Schumacher, Günter, Dr. et al**
**c/o Bayer AG Konzernverwaltung RP**
**Patentabteilung**
**D-5090 Leverkusen 1 Bayerwerk(DE)**

(54) **2,5-Dihydropyrrole.**

(57) Offenbart werden neue 2,5-Dihydropyrrole der Formel (I)

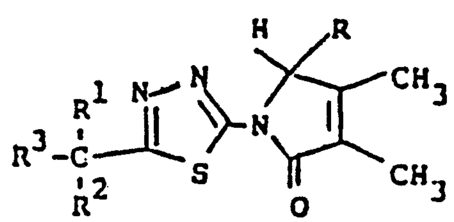

(I)

und die Verwendung der neuen Verbindungen als selektive Herbizide.

EP 0 286 816 A1

## 2,5-Dihydropyrrole

Die vorliegende Erfindung betrifft neue 2,5-Dihydropyrrole, mehrere Verfahren zu ihrer Herstellung und ihre Verwendung als selektive Herbizide.

Es wurde bereits offenbart, daß bestimmte 2,5-Dihydropyrrole als Mittel zur Schädlingsbekämpfung geeignet sind (siehe die JP-OS 23965/1978, die der DE-OS 27 35 841 oder der US-PS 4 138 243 entspricht).

Nunmehr wurden neue 2,5-Dihydropyrrole der Formel (I)

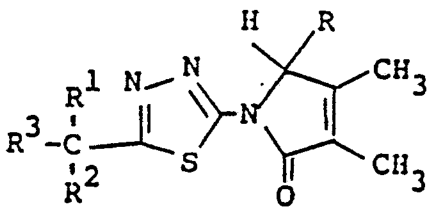

(I)

gefunden, in der

$R^1$ Wasserstoff oder eine Alkyl-Gruppe mit 1 bis 4 Kohlenstoff-Atomen bezeichnet,

$R^2$ und $R^3$ jeweils eine Alkyl-Gruppe mit 1 bis 4 Kohlenstoff-Atomen oder eine Halogenoalkyl-Gruppe mit 1 bis 4 Kohlenstoff-Atomen bezeichnen oder gemeinsam eine $C_{2-6}$-Alkylen-Gruppe bilden können, die durch Halogen substituiert sein kann, und

R eine Hydroxy-Gruppe, eine Mercapto-Gruppe, ein Halogen-Atom, eine Aminogruppe oder eine Gruppe der Formel -O-W bezeichnet, in der W eine Alkylcarbonyl-, eine Halogenalkylcarbonyl-, eine Alkoxycarbonyl-oder eine Halogenalkoxycarbonyl-Gruppe mit 1 bis 4 Kohlenstoff-Atomen in der Alkyl-Gruppe, eine Alkylsulfonyl-oder eine Halogenalkylsulfonyl-Gruppe mit jeweils 1 bis 4 Kohlenstoffatomen in der Alkyl-Gruppe oder eine Di-$C_1$-$C_4$-alkylaminosulfonyl-Gruppe darstellt.

2,5-Dihydropyrrole der Formel (I) werden erhalten, wenn

(a) in dem Fall, in dem R in der Formel (I) Hydroxy ist, Verbindungen der Formel (II)

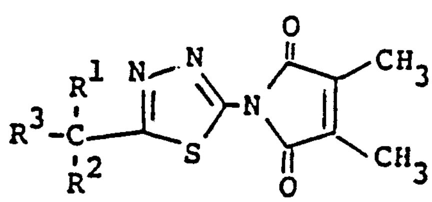

(II)

,

in der

$R^1$, $R^2$ und $R^3$ die im Vorstehenden angegebenen Bedeutungen haben, mit einem Reduktionsmittel in Gegenwart inerter Lösungsmittel umgesetzt werden oder

b) in dem Fall, in dem R in der Formel (I) ein Halogen-Atom ist, 2,5-Dihydropyrrole der Formel (Ia)

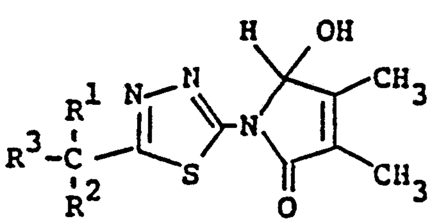

(Ia)

,

in der

$R^1$, $R^2$ und $R^3$ die im Vorstehenden angegebenen Bedeutungen haben, mit einem Halogenierungsmittel in Gegenwart inerter Lösungsmittel umgesetzt werden oder

(c) in dem Fall, in dem R in der Formel (I) die Gruppe -O-W ist, 2,5-Dihydropyrrole der oben bezeichneten Formel (Ia) mit Verbindungen der Formel (III)

W-Hal (III),

in der W die oben angegebenen Bedeutungen hat und Hal ein Halogen bezeichnet, oder mit Verbindungen der Formel (IV)

W-O-W (IV),

in der W die oben angegebenen Bedeutungen hat, in Gegenwart inerter Lösungsmittel, gegebenenfalls in Gegenwart säurebindender Mittel, umgesetzt werden oder

d) in dem Fall, in dem R in der Formel (I) eine Mercapto-Gruppe ist, 2,5-Dihydropyrrole der Formel (Ib)

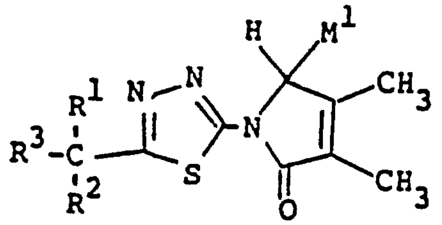

(Ib) ,

in der

$R^1$, $R^2$ und $R^3$ die im Vorstehenden angegebenen Bedeutungen haben und $M^1$ Halogen bezeichnet, mit Kaliumhydrogensulfid, Thioharnstoff oder N,N-Di-methylthioformamid in Gegenwart inerter Lösungsmittel umgesetzt werden.

Die neuen 2,5-Dihydropyrrole zeigen potente und selektive herbizide Eigenschaften.

Überraschenderweise zeigen die erfindungsgemäßen 2,5-Dihydropyrrole eine wesentlich größere selektive herbizide Wirkung als diejenigen, die aus dem Stand der Technik, beispielsweise aus der oben angegebenen JP-OS 23965/1978, bekannten, und weisen gleichzeitig auch eine gute Verträglichkeit mit Nutzpflanzen auf.

Unter den 2,5-Dihydropyrrolen gemäß der Erfindung sind bevorzugte Verbindungen der Formel (I) diejenigen, in denen

$R^1$ Methyl oder Ethyl bezeichnet,

$R^2$ und $R^3$ jeweils Methyl, Ethyl oder eine chloro-oder fluorosubstituierte $C_{1-2}$-Alkyl-Gruppe bezeichnen und

R Hydroxy, Mercapto, Chloro, Acetyloxy, Methansulfonyloxy, Trifluoracetyloxy, Chloracetyloxy, Trifluormethylsulfonyloxy oder Dimethylaminosulfonyloxy darstellt.

Speziell erwähnt sei die nachstehende Verbindung: N-(5-tert-Butyl-1,3,4-thiadiazol-2-yl)-3,4-dimethyl-2-hydroxy-5-oxo-2,5-dihydropyrrol.

Wenn in dem Verfahren a) beispielsweise N-(5-tert-Butyl-1,3,4-thiadiazol-2-yl)-2,3-dimethylmaleinimid und Natriumborhydrid als Ausgangsstoffe eingesetzt werden, kann der Reaktionsverlauf durch die folgende Gleichung dargestellt werden:

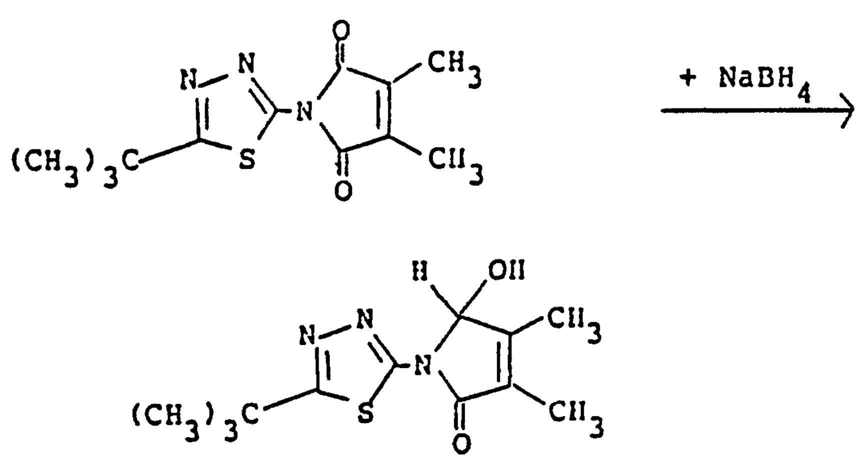

Wenn in dem Verfahren b) beispielsweise N-(5-tert-Butyl-1,3,4-thiadiazol-2-yl)-3,4-dimethyl-2-hydroxy-5-oxo-2,5-dihydropyrrol und Thionylchlorid als Ausgangsstoffe eingesetzt werden, kann der Reaktionsverlauf durch die folgende Gleichung dargestellt werden:

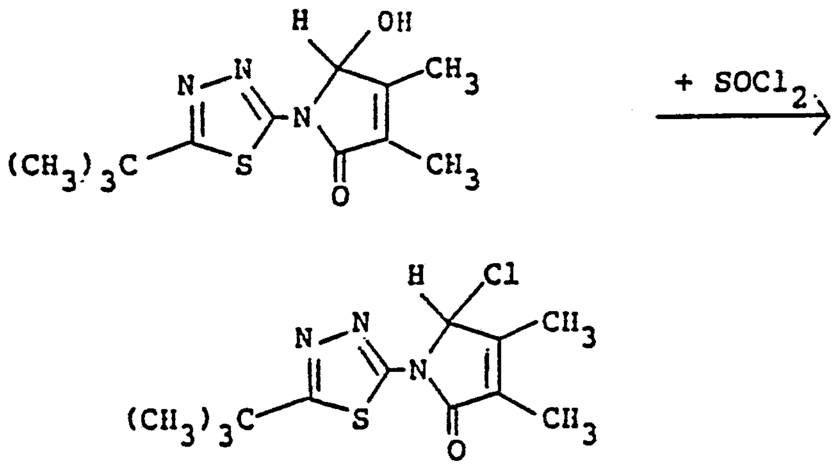


Wenn in dem Verfahren c) beispielsweise N-(5-tert-Butyl-1,3,4-thiadiazol-2-yl)-3,4-dimethyl-2-hydroxy-5-oxo-2,5-dihydropyrrol und Acetylchlorid als Ausgangsstoff eingesetzt werden, kann der Reaktionsverlauf durch die folgende Gleichung dargestellt werden:

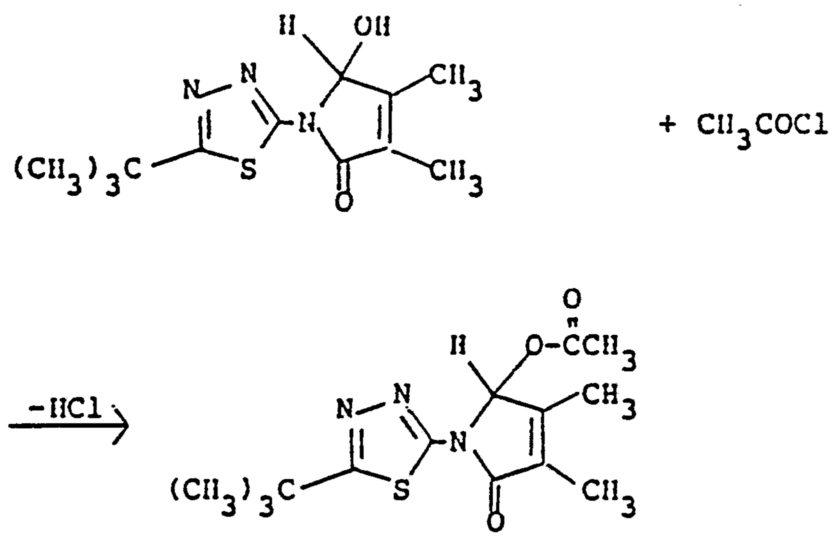


Wenn in dem Verfahren d) beispielsweise N-(5-tert-Butyl-1,3,4-thiadiazol-2-yl)-3,4-dimethyl-2-chloro-5-oxo-2,5-dihydropyrrol und N,N-Dimethylthioformamid als Ausgangsstoffe eingesetzt werden, kann der Reaktionsverlauf durch die folgende Gleichung dargestellt werden:

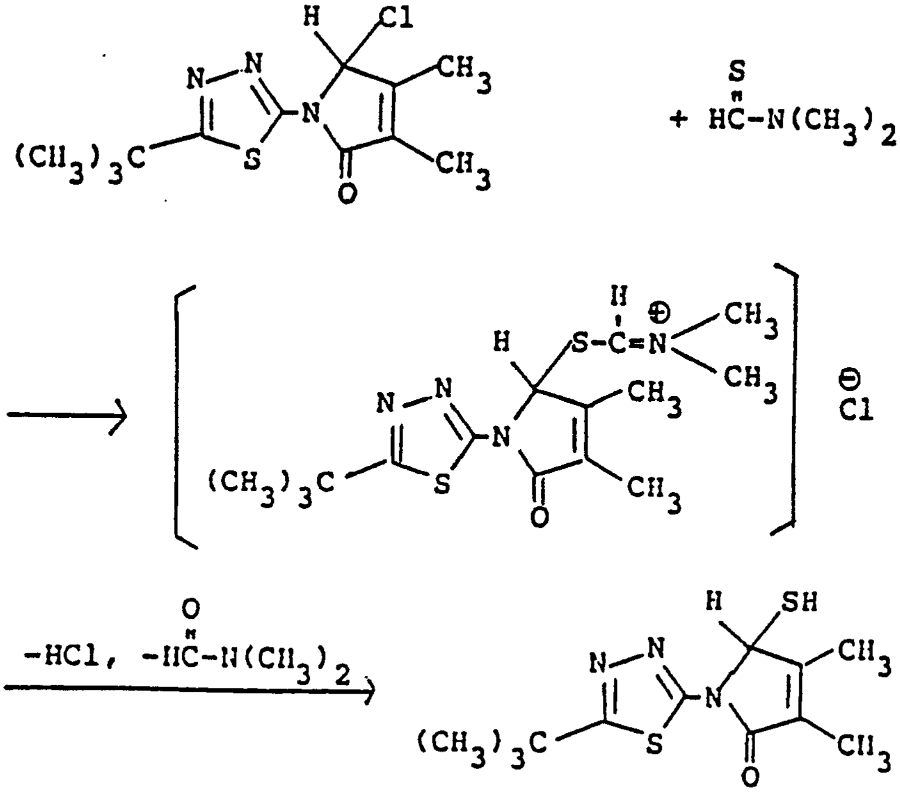


In dem Verfahren a) bezeichnen die Ausgangs-Verbindungen der Formel (II) Verbindungen auf der Basis der oben gegebenen Definitionen für $R^1$, $R^2$ und $R^3$, vorzugsweise Verbindungen auf der Basis der oben gegebenen bevorzugten Definitionen.

Die Verbindungen der Formel (II) sind neue Verbindungen und können erhalten werden, wenn Verbindungen der Formel (V)

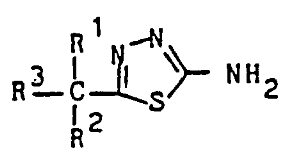

(V)

,

in der $R^1$, $R^2$ und $R^3$ die oben angegebenen Bedeutungen haben, mit Verbindungen der Formel (VI)

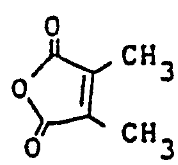

(VI)

oder, auf 1 mol der Verbindung der Formel (V), mit 2 mol Maleinsäureanhydrid umgesetzt werden.

Die Verbindungen der Formel (V) sind bekannt. Ein besonderes Beispiel für dieselben ist 2-Amino-5-tert-butyl-1,3,4-thiadiazol, das in Can. J. Chem., Band 37, Seiten 1121-1123 (1959) beschrieben ist.

2,3-Dimethylmaleinsäureanhydrid der Formel (VI) ist, beispielsweise, beschrieben in der JP-OS 23965/1978. Ein spezielles Beispiel für die Verbindungen der Formel (II) ist N-(5-tert-Butyl-1,3,4-thiadiazol-2-yl)dimethylmaleinimid.

Metallhydride wie Natriumborhydrid und Lithiumaluminiumhydrid lassen sich als Beispiele für das Reduktionsmittel in dem Verfahren a) anführen.

Die in dem Verfahren b) eingesetzten Ausgangs-Verbindungen der Formel (Ia) zählen zu den Verbindungen der Formel (I), die mittels des Verfahrens a) hergestellt werden können.

Thionylchlorid läßt sich als Beispiel für die in dem Verfahren b) verwendeten Halogenierungsmittel anführen.

In dem Verfahren c) bezeichnen die Ausgangs-Verbindungen der Formeln (III) oder (IV) Verbindungen auf der Basis der Definitionen für W und Hal, und vorzugsweise bezeichnen W eine Acetyl-Gruppe und Hal ein Chlor-Atom.

Acetylchlorid läßt sich als spezielles Beispiel für die wohlbekannten Verbindungen der Formel (III) nennen.

Die Verbindungen der Formel (IV) sind ebenfalls wohlbekannt, und für sie läßt sich als spezielles Beispiel Essigsäureanhydrid zitieren.

Sämtliche inerten Lösungsmittel sind geeignete Verdünnungsmittel, die bei der praktischen Durchführung des Verfahrens a) verwendet werden.

Beispiele für diese Verdünnungsmittel umfassen Wasser, Ether (z.B. Dioxan und Tetrahydrofuran), Alkohole (z.B. Methanol, Ethanol, Isopropanol, Butanol und Ethylenglycol) sowie Säureamide (z.B. Dimethylformamid).

Das Verfahren a) kann bei Temperaturen innerhalb eines Bereichs von beträchlicher Breite praktisch durchgeführt werden, beispielsweise bei etwa -20 °C bis etwa 80 °C, vorzugsweise bei etwa 0 °C bis etwa 30 °C.

Die Reaktion wird zweckmäßigerweise unter Atmosphärendruck durchgeführt, kann jedoch auch unter erhöhtem oder vermindertem Druck durchgeführt werden.

Bei der Durchführung des Verfahrens a) können die gewünschten Verbindungen der Formel (I) in einfacher Weise durch Reduzieren der Verbindungen der Formel (II) mit dem oben beispielhaft genannten Reduktionsmittel erhalten werden, wie in einem nachstehend angegebenen Arbeitsbeispiel gezeigt wird.

Bei der praktischen Durchführung des Verfahrens b) seien Kohlenwasserstoff (z.B. Benzol, Toluol und Xylol) und halogenhaltige Kohlenwasserstoffe (z.B. Methylenchlorid, Chloroform, Kohlenstofftetrachlorid, 1,2-Dichlorethan und Chlorbenzol) als geeignete Verdünnungsmittel genannt.

Das Verfahren b) kann bei Temperaturen innerhalb eines Bereichs von beträchtlicher Breite durchgeführt werden, beispielsweise bei etwa -20 °C bis etwa 150 °C, vorzugsweise bei etwa 0 °C bis etwa 80 °C.

Die Reaktion wird zweckmäßigerweise unter Atmosphärendruck durchgeführt, kann jedoch auch unter erhöhtem oder vermindertem Druck durchgeführt werden.

Bei der Durchführung des Verfahrens b) können die gewünschten Verbindungen der Formel (I) in einfacher Weise beispielsweise durch Chlorieren der Verbindungen der Formel (Ia) mit Thionylchlorid erhalten werden.

Für die praktische Durchführung des Verfahrens c) lassen sich Ether (z.B. Dioxan und Tetrahydrofuran), Säureamide (wie Dimethylformamid, Dimethylacetamid und Hexamethylphosphorsäuretriamid) und Sulfoxide (wie Dimethylsulfoxid) als geeignete Verdünnungsmittel anführen.

Das Verfahren c) kann in Gegenwart eines säurebindenden Mittels durchgeführt werden. Zu Beispielen für ein solches säurebindendes Mittel zählen Hydride, Hydroxide, Carbonate, Hydrogencarbonate und Alkoholate von Alkalimetallen sowie tertiäre Amine wie Triethylamin, Diethylanilin, Pyridin, 4-Dimethylaminopyridin, 1,8-Diazabicyclo[5.4.0]undecen (DBU) und 1,4-Diazabicyclo[2.2.2]octan (DABCO).

Das Verfahren c) kann bei Temperaturen innerhalb eines Bereichs von beträchlicher Breite durchgeführt werden, beispielsweise bei etwa 50 °C bis etwa 200 °C, vorzugsweise bei etwa 80 °C bis etwa 160 °C.

Die Reaktion wird zweckmäßigerweise unter Atmosphärendruck durchgeführt, kann jedoch auch unter erhöhtem oder vermindertem Druck durchgeführt werden.

Bei der Durchführung des Verfahrens c) können die gewünschten Verbindungen der Formel (I) beispielsweise dadurch erhalten werden, daß 1 mol der Verbindungen der Formel (Ia) mit 1 bis etwa 1,5 mol der Verbindungen der Formel (III) oder (IV) in einem inerten Lösungsmittel, gegebenenfalls in Gegenwart des säurebindenden Mittels, umgesetzt werden.

Für die praktische Durchführung des Verfahrens d) lassen sich Alkohole (z.B. Methanol, Ethanol, Isopropanol), aromatische Kohlenwasserstoffe (z.B. Benzol, Toluol, Xylol), Amide (z.B. N,N-Dimethylformamid, N,N-Dimethylacetamid, 1-Methyl-2-pyrrolidinon) und Sulfoxide (z.B. Dimethylsulfoxid, Sulfolan) als geeignete Verdünnungsmittel anführen.

Das Verfahren d) kann bei Temperaturen innerhalb eines Bereichs von beträchtlicher Breite durchgeführt werden, beispielsweise bei etwa 50 °C bis etwa 160 °C, vorzugsweise bei etwa 60 °C bis etwa 130 °C.

Die Reaktion wird zweckmäßigerweise unter Atmosphärendruck durchgeführt, kann jedoch auch unter erhöhtem oder vermindertem Druck durchgeführt werden.

Bei der Durchführung des Verfahrens d) können die gewünschten Verbindungen der Formel (I) beispielsweise dadurch erhalten werden, daß 1 mol der Verbindungen der Formel (Ib) mit 1 bis etwa 1,2

mol N,N-Dimethylthioformamid in Gegenwart der oben genannten inerten Lösungsmittel umgesetzt werden.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendet werden:

Dikotyledonen-Unkräuter der Gattungen:
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen:
Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

Monkotyledonen-Unkräuter der Gattungen:
Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:
Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegeländen und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Präparate wie Lösungen, Emulsionen, Suspensionen, Pulver, Stäubemittel, Schäume, Pasten, lösliche Pulver, Granulat, Aerosole, mit dem Wirkstoff imprägnierte natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs-und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder

Glycol sowie deren Ether und Ester, Ketone wie Aceton, Methylethylketon, Methylisobutylketon oder Cyclohexanon, oder stark polare Lösungsmittel wie Dimethylformamid oder Dimethysulfoxid, sowie auch Wasser.

Unter verflüssigten gasförmigen Verdünnungsmitteln oder Trägern sind Flüssigkeiten zu verstehen, die bei normaler Temperatur und unter normalen Druck gasförmig sein würden, beispielsweise Aerosol-Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nicht-ionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate auch zur Unkrautbekämpfung als Mischungen mit anderen Herbiziden eingesetzt werden, wobei Fertigpräparate oder Tankmischungen möglich sind.

Mischungen mit anderen aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können entweder vor oder nach dem Auflaufen der Pflanzen zur Anwendung gelangen.

Sie können auch vor der Einsaat in den Boden eingearbeitet werden. Insbensondere werden sie nach dem Auflaufen der Pflanzen angewandt.

Die eingesetzten Mengen der aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen 0,001 und 4 kg/ha, vorzugsweise zwischen 0,05 und 2 kg/ha.

Die Herstellung und Verwendung der erfindungsgemäßen Verbindungen sind den folgenden Beispielen zu entnehmen.

<u>Herstellungsbeispiele</u>

Beispiel 1

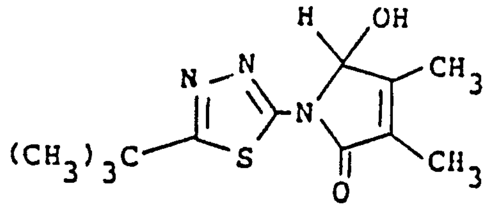

(Verbindung Nr. 1)

Natriumborhydrid (0,07 g) wurde bei Raumtemperatur zu einer methanolischen Lösung (100 ml) von N-(5-tert-Butyl-1,3,4-thiadiazol-2-yl)-2,3-dimethylmaleinimid (0,95 g) hinzugefügt, und die Mischung wurde 3 h gerührt. Nach der Reaktion wurde Eisessig (0,5 ml) hinzugefügt, und das Lösungsmittel wurde unter vermindertem Druck abgedampft. Der Rückstand wurde mit Wasser (30 ml) versetzt, und Kaliumcarbonat wurde hinzugegeben, um die Mischung alkalisch zu machen. Die Mischung wurde mit Dichloromethan (2 × 50 ml) extrahiert. Die Extrakte wurden über wasserfreiem Magnesiumsulfat getrocknet, und das Lösungsmittel wurde unter vermindertem Druck abgedampft. Die resultierenden rohen Kristalle wurden mit einer kleinen Menge Diethylether gewaschen, wonach das gewünschte N-(5-tert-Butyl-1,3,4-thiadiazol-2-yl)-3,4-dimethyl-2-hydroxy-5-oxo-2,5-dimethylpyrrol (0,74 g) erhalten wurde; Schmp. 179-182 °C.

Die nachstehende Tabelle 1 zeigt Verbindungen der Erfindung, die nach der oben beschriebenen Arbeitsweise erhalten werden.

Tabelle 1

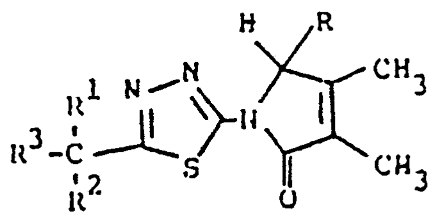

| Verbindung Nr. | $R^1$<br>$R^3-C-$<br>$R^2$ | R | Physikalische Eigenschaften |
|---|---|---|---|
| 2 | $-C(CH_3)_3$ | $-OCOCH_3$ | $n_D^{20}$ 1.5290 |
| 3 | $-C(CH_3)_3$ | -Cl | Schmp. 78-80°C |
| 4 | $C_2H_5$<br>$-C(CH_3)_2$ | -OH | Schmp. 128-131°C |
| 5 | $CH_3$<br>$-C(C_2H_5)_2$ | -OH | Schmp. 128-130°C |
| 6 | $CH_2Cl$<br>$-C(CH_3)_2$ | -OH | Schmp. 155-156°C |
| 7 | $CH_2F$<br>$-C(CH_3)_2$ | -OH | Schmp. 180-181°C |
| 8 | $CH_3$<br>$-C(CH_2Cl)_2$ | -OH | |
| 9 | $CH_3$<br>$-C(CH_2F)_2$ | -OH | |
| 10 | $CHCl_2$<br>$-C(CH_3)_2$ | -OH | |
| 11 | $CHF_2$<br>$-C(CH_3)_2$ | -OH | |
| 12 | $C(CH_3)_3$<br>$-C(CH_3)_2$ | -OH | |

Tabelle 1 - Fortsetzung

| Verbindung Nr. | $R^1$<br>$R^3-C-$<br>$R^2$ | R | Physikalische Eigenschaften |
|---|---|---|---|
| 13 | 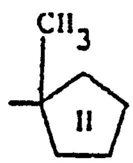 | -OH | |
| 14 | 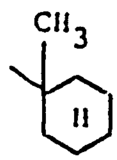 | -OH | |
| 15 | 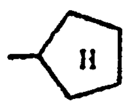 | -OH | Schmp.197-198°C |
| 16 | $-C(CH_3)_3$ | -SH | |
| 17 | $C_2H_5$<br>$-C(CH_3)_2$ | -Cℓ | |

| Verbindung Nr. | 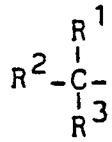 | R | physikalische Eigenschaften |
|---|---|---|---|
| 18 | 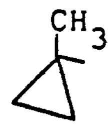 | OH | |
| 19 | 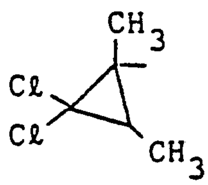 | OH | |
| 20 | $(CH_3)_3C-$ | $OCOCH_2Cl$ | Schmp.: 116 - 118°C |
| 21 | $(CH_3)_3C-$ | $OCOCF_3$ | viskoses Öl |
| 22 | $(CH_3)_3C-$ | $OSO_2CH_3$ | Schmp.: 106 - 110°C |
| 23 | $(CH_3)_3C-$ | $OSO_2CF_3$ | viskoses Öl |
| 24 | $(CH_3)_3C-$ | $OSO_2N(CH_3)_2$ | Schmp.: 135 - 140°C |
| 25 | $(CH_3)_3C-$ | Br | |
| 26 | $(CH_3)_3C-$ | $NH_2$ | |
| 27 | $(CH_3)_2CH-$ | OH | Schmp.: 170 - 173°C |
| 28 | $(CH_3)_2CH-$ | Cl | Schmp.: 76 - 78.5°C |
| 29 | $CH_3CH_2CH-$<br>$CH_3$ | OH | Schmp.: 130 - 131.5°C |
| 30 | $CH_3CH_2CH-$<br>$CH_3$ | Cl | $n_D^{20}$ 1.5460 |
| 31 | $(CH_3)_3C-$ | $OCOOCH_3$ | |
| 32 | $(CH_3)_3C-$ | $OCOOCH_2CF_3$ | |

Beispiel 2

Synthese eines Zwischenprodukts:

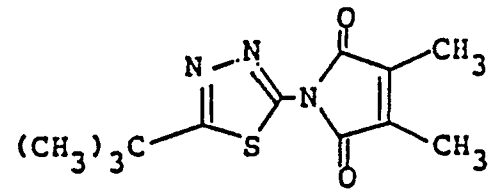

2-Amino-5-tert-butyl-1,3,4-thiadiazol (1,57 g) und 2,3-Dimethylmaleinsäureanhydrid (1,51 g) wurden 5 h in Eisessig (30 ml) zum Rückfluß erhitzt. Nach der Reaktion wurden die Essigsäure und der Überschuß an 2,3-Dimethylmaleinsäureanhydrid abgedampft. Der Rückstand wurde in Dichloromethan (50 ml) gelöst, und die Lösung wurde mit einer 10-proz. wäßrigen Kaliumcarbonat-Lösung (20 ml) gewaschen und über wasserfreiem Magnesiumsulfat getrocknet. Das Lösungsmittel wurde abgedampft, wonach das gewünschte N-(5-tert-Butyl-1,3,4-thiadiazol-2-yl)-2,3-dimethylmaleinimid (2,41 g) erhalten wurde; Schmp. 87-91 °C.

Biologische Tests

Vergleichsverbindungen:

E-1:

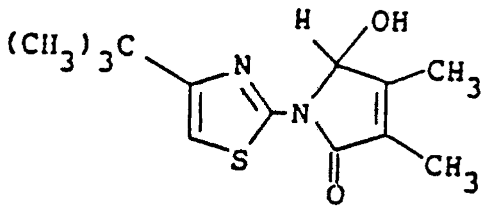

(beschrieben in der JP-OS 23965/1978).

E-2:

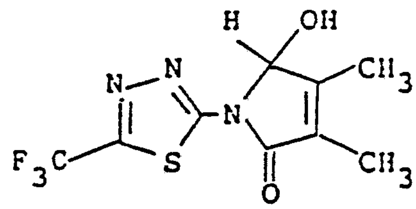

(beschrieben in der JP-OS 23965/1978).

E-3:

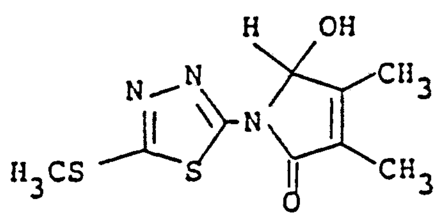

(beschrieben in der JP-OS 23965/1978).

Beispiel 3

Test mittels Bodenbehandlung vor dem Auflaufen:

Herstellung eines Wirkstoff-Präparats

Träger: 5 Gewichtsteile Aceton;

Emulgator: 1 Gewichtsteil Benzyloxypolyglycolether.

Ein Wirkstoff-Präparat in Form eines emulgierbaren Konzentrats wird erhalten durch Vermischen von 1 Gewichtsteil der aktiven Verbindung mit den oben angegebenen Mengen des Trägers und des Emulgators. Eine vorher festgelegte Menge des Präparats wird mit Wasser verdünnt, um eine Test-Chemikalie zu erhalten.

Test-Verfahren

In einem Gewächshaus wurden Mais-Samen in Töpfe (500 $cm^2$), die mit Ackererde gefüllt waren, eingesät, und Erde, die Samen von Gänsefuß (Chenopodium album L.), Hühnerhirse (Echinocloa crus-galli) und wildem grauen Amaranth (Amaranthus lividus L.) enthielt, wurde über die Mais-Samen in einer Tiefe von 1 cm gestreut.

Einen Tag nach der Einsaat wurde eine vorher festgelegte Menge der Test-Chemikalie gleichmäßig über die Oberflächenschicht des Bodens in jedem der Test-Töpfe gesprüht.

Vier Wochen nach dem Sprühen wurden die herbizide Wirkung und der Grad der Phytotoxizität gegenüber den Nutzpflanzen (Mais) geprüft und mit Hilfe der folgenden Skalen von 0 bis 5 bewertet:

Die herbizide Wirkung wurde als prozentuales Verhältnis der Unkrautvernichtung, bezogen auf eine unbehandelte Fläche, nach der folgenden Skala bewertet:

| | |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |

Die Phytotoxizität gegenüber Mais wurde als Phytotoxizitätsrate, bezogen auf die unbehaldelte Fläche, nach der folgenden Skala bewertet:

| | |
|---|---|
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Test-Ergebnisse sind anhand typischer Beispiele in Tabelle 2 aufgeführt.

Tabelle 2

| Verbindung Nr. | Wirkstoff-Menge kg/ha | Herbizide Wirkung A | B | C | Phytotoxizität gegen Mais |
|---|---|---|---|---|---|
| 1 | 0,5 | 5 | 5 | 5 | 0 |
| | 0,25 | 5 | 5 | 4 | 0 |
| Vergleichs-Verbindungen | | | | | |
| E-1 | 0,5 | 1 | 1 | 0 | 0 |
| | 0,25 | 0 | 0 | 0 | 0 |
| E-2 | 0,5 | 1 | 2 | 0 | 0 |
| | 0,25 | 0 | 1 | 0 | 0 |
| E-3 | 0,5 | 1 | 1 | 0 | 0 |
| | 0,25 | 0 | 0 | 0 | 0 |

A: Wilder grauer Amaranth
B: Gänsefuß
C: Hühnerhirse

Beispiel 4

Test auf höher gelegenem Ackerboden mittels Laub-Behandlung:

In einem Gewächshaus wurden Mais-Samen in Töpfe (500 $cm^2$), die mit Ackererde gefüllt waren, eingesät, und Erde, die Samen von wildem grauen Amaranth und Gänsefuß enthielt, wurde in einer Tiefe von 1 cm darüber gestreut.

Nach der Einsaat wurden die Pflanzen 14 Tage angezogen, und eine vorher festgelegte Menge einer Test-Chemikalie, die wie in Beispiel 3 hergestellt worden war, wurde gleichmäßig über die Pflanzen in den Töpfen gesprüht.

Vier Wochen nach dem Sprühen wurden die herbizide Wirkung und die Phytotoxizität gegenüber den Nutzpflanzen (Mais) anhand der gleichen Bewertungsskalen wie in Beispiel 3 geprüft. Die Ergebnisse sind anhand typischer Beispiele in Tabelle 3 aufgeführt.

Tabelle 3

| Verbindung Nr. | Wirkstoff-Menge kg/ha | Herbizide Wirkung A | Herbizide Wirkung B | Phytotoxizität gegen Mais |
|---|---|---|---|---|
| 1 | 0,5 | 5 | 5 | 0 |
| | 0,25 | 5 | 5 | 0 |
| Vergleichs-Verbindungen | | | | |
| E-1 | 0,5 | 2 | 2 | 0 |
| | 0,25 | 1 | 1 | 0 |
| E-2 | 0,5 | 3 | 3 | 0 |
| | 0,25 | 1 | 2 | 0 |
| E-3 | 0,5 | 2 | 2 | 0 |
| | 0,25 | 1 | 1 | 0 |

A und B: die gleichen, wie sie in der Fußnote zu Tabelle 2 bezeichnet sind.

**Ansprüche**

1. 2,5-Dihydropyrrole der Formel (I)

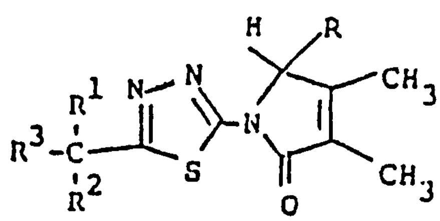

(I)

in der

$R^1$ Wasserstoff oder eine Alkyl-Gruppe mit 1 bis 4 Kohlenstoff-Atomen bezeichnet,

$R^2$ und $R^3$ jeweils eine Alkyl-Gruppe mit 1 bis 4 Kohlenstoff-Atomen oder eine Halogenoalkyl-Gruppe mit 1 bis 4 Kohlenstoff-Atomen bezeichnen oder gemeinsam eine $C_{2-6}$-Alkylen-Gruppe bilden können, die durch Halogen substituiert sein kann, und

R eine Hydroxyl-Gruppe, eine Mercapto-Gruppe, ein Halogen-Atom, eine Amino-Gruppe oder eine Gruppe der Formel -O-W bezeichnet, in der W eine Alkylcarbonyl-, eine Halogenalkylcarbonyl-, eine Alkoxycarbonyl-oder eine Halogenalkoxycarbonyl-Gruppe mit 1 bis 4 Kohlenstoffatomen in der Alkyl-Gruppe, eine Alkylsulfonyl-oder eine Halogenalkylsulfonyl-Gruppe mit jeweils 1 bis 4 Kohlenstoffatomen in der Alkyl-Gruppe oder eine Di-$C_1$-$C_4$-alkylaminosulfonyl-Gruppe darstellt.

2. Verbindungen nach Anspruch 1, dadurch gekennzeichnet, daß
$R^1$ Methyl oder Ethyl bezeichnet,
$R^2$ und $R^3$ jeweils Methyl, Ethyl oder eine chloro-oder fluorosubstituierte $C_{1\,2}$-Alkyl-Gruppe bezeichnen und
R Hydroxy, Mercapto, Chloro, Acetyloxy, Methansulfonyloxy, Trifluoracetyloxy, Chloracetyloxy, Trifluormethylsulfonyloxy oder Dimethylaminosulfonyloxy darstellt.

3. N-(5-tert-Butyl-1,3,4-thiadiazol-2-yl)-3,4-dimethyl-2-hydroxy-5-oxo-2,5-dihydropyrrol nach den Ansprüchen 1 und 2, dargestellt durch die Formel

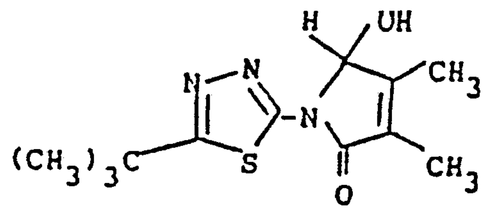

.

4. Verfahren zur Herstellung von 2,5-Dihydropyrrolen der Formel (I)

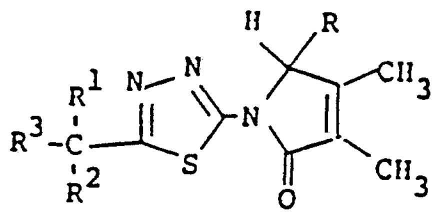

(I)

,

in der
$R^1$ Wasserstoff oder eine Alkyl-Gruppe mit 1 bis 4 Kohlenstoff-Atomen bezeichnet,
$R^2$ und $R^3$ jeweils eine Alkyl-Gruppe mit 1 bis 4 Kohlenstoff-Atomen oder eine Halogenoalkyl-Gruppe mit 1 bis 4 Kohlenstoff-Atomen bezeichnen oder gemeinsam eine $C_{2\,6}$-Alkylen-Gruppe bilden können, die durch Halogen substituiert sein kann, und
R eine Hydroxy-Gruppe, eine Mercapto-Gruppe, ein Halogen-Atom, eine Amino-Gruppe oder eine Gruppe der Formel -O-W bezeichnet, in der W eine Alkylcarbonyl-, eine Halogenalkylcarbonyl-, eine Alkoxycarbonyl-oder eine Halogenalkoxycarbonyl-Gruppe mit 1 bis 4 Kohlenstoffatomen in der Alkyl-Gruppe, eine Alkylsulfonyl-oder eine Halogenalkylsulfonyl-Gruppe mit jeweils 1 bis 4 Kohlenstoffatomen in der Alkyl-Gruppe oder eine Di-$C_1$-$C_4$-alkylaminosulfonyl-Gruppe, darstellt,
dadurch gekennzeichnet, daß
a) in dem Fall, in dem R in der Formel (I) Hydroxy ist, Verbindungen der Formel (II)

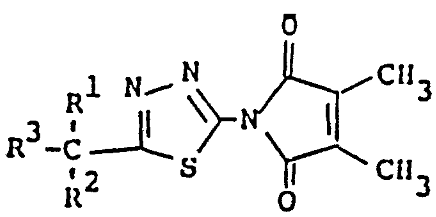

(II)

,

in der
$R^1$, $R^2$ und $R^3$ die im Vorstehenden angegebenen Bedeutungen haben, mit einem Reduktionsmittel in Gegenwart inerter Lösungsmittel umgesetzt werden oder
b) in dem Fall, in dem R in der Formel (I) ein Halogen-Atom ist, 2,5-Dihydropyrrole der Formel (Ia)

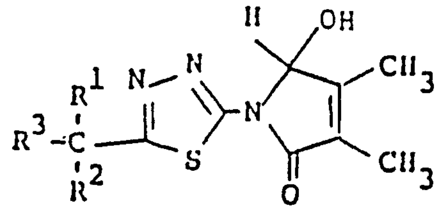

(Ia) ,

in der

$R^1$, $R^2$ und $R^3$ die im Vorstehenden angegebenen Bedeutungen haben, mit einem Halogenierungsmittel in Gegenwart inerter Lösungsmittel umgesetzt werden oder

c) in dem Fall, in dem R in der Formel (I) die Gruppe -O-W ist, 2,5-Dihydropyrrole der oben bezeichneten Formel (Ia) mit Verbindungen der Formel (III)

W-Hal (III),

in der W die oben angegebenen Bedeutungen hat und Hal ein Halogen bezeichnet, oder mit Verbindungen der Formel (IV)

W-O-W (IV),

in der W die oben angegebenen Bedeutungen hat, in Gegenwart inerter Lösungsmittel, gegebenenfalls in Gegenwart säurebindender Mittel, umgesetzt werden oder

d) in dem Fall, in dem R in der Formel (I) eine Mercapto-Gruppe ist, 2,5-Dihydropyrrole der Formel (Ib)

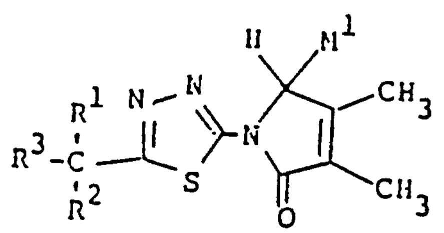

(Ib) ,

in der

$R^1$, $R^2$ und $R^3$ die im Vorstehenden angegebenen Bedeutungen haben und $M^1$ Halogen bezeichnet, mit Kaliumhydrogensulfid, Thioharnstoff oder N,N-Dimethylthioformamid in Gegenwart inerter Lösungsmittel umgesetzt werden.

5. Herbizide Mittel, dadurch gekennzeichnet, daß sie wenigstens ein 2,5-Dihydropyrrol der Formel (I) nach den Ansprüchen 1 bis 4 enthalten.

6. Verfahren zur Bekämpfung von Unkräutern, dadurch gekennzeichnet, daß 2,5-Dihydropyrrole der Formel (I) nach den Ansprüchen 1 bis 4 auf Unkräuter und/oder deren Lebensraum zur Einwirkung gebracht werden.

7. Verwendung von 2,5-Dihydropyrrole der Formel (I) nach den Ansprüchen 1 bis 4 zur Bekämpfung von Unkräutern.

8. Verfahren zur Herstellung herbizider Mittel, dadurch gekennzeichnet, daß 2,5-Dihydropyrrole der Formel (I) nach den Ansprüchen 1 bis 4 mit Streckmitteln und/oder grenzflächenaktiven Mitteln vermischt werden.

9. Verbindungen der Formel (II)

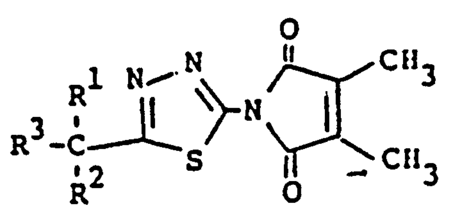

(II) ,

in der

$R^1$ Wasserstoff oder eine Alkyl-Gruppe mit 1 bis 4 Kohlenstoff-Atomen bezeichnet und

$R^2$ und $R^3$ jeweils eine Alkyl-Gruppe mit 1 bis 4 Kohlenstoff-Atomen oder eine Halogenoalkyl-Gruppe mit 1 bis 4 Kohlenstoff-Atomen bezeichnen oder gemeinsam eine $C_{2-6}$-Alkylen-Gruppe bilden können, die durch Halogen substituiert sein kann.

10. Verfahren zur Herstellung von Verbindungen der Formel (II)

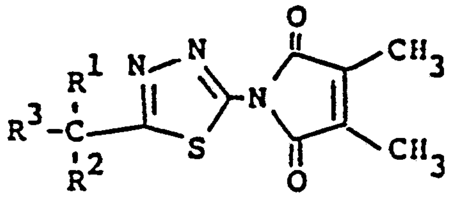

(II)

,

in der

$R^1$ Wasserstoff oder eine Alkyl-Gruppe mit 1 bis 4 Kohlenstoff-Atomen bezeichnet und

$R^2$ und $R^3$ jeweils eine Alkyl-Gruppe mit 1 bis 4 Kohlenstoff-Atomen oder eine Halogenoalkyl-Gruppe mit 1 bis 4 Kohlenstoff-Atomen bezeichnen oder gemeinsam eine $C_{2-6}$-Alkylen-Gruppe bilden können, die durch Halogen substituiert sein kann,

dadurch gekennzeichnet, daß Verbindungen der Formel (V)

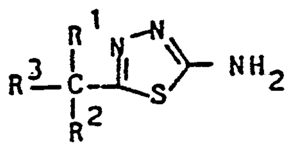

(V)

,

in der $R^1$, $R^2$ und $R^3$ die oben angegebenen Bedeutungen haben, mit Verbindungen der Formel (VI)

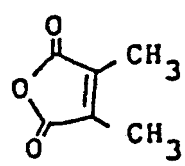

(VI)

oder, auf 1 mol der Verbindung der Formel (V), mit 2 mol Maleinsäureanhydrid umgesetzt werden.

Europäisches Patentamt

# EUROPÄISCHER RECHERCHENBERICHT

Nummer der Anmeldung

EP 88 10 3122

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,Y | FR-A-2 400 013 (CIBA-GEIGY)<br>* Insgesamt; Seite 1, Zeilen 36,37; Seite 2, Zeilen 1-12 * | 1-10 | C 07 D 417/04<br>A 01 N 43/82 |
| Y | FR-A-2 263 247 (BAYER AG)<br>* Insgesamt * | 1-8 | |
| Y | FR-A-2 347 364 (BAYER AG)<br>* Insgesamt * | 1-8 | |
| X | FR-A-2 278 696 (CIBA-GEIGY)<br>* Insgesamt * | 9,10 | |
| | | | RECHERCHIERTE SACHGEBIETE (Int. Cl.4) |
| | | | C 07 D 417/00 |

Der vorliegende Recherchenbericht wurde für alle Patentansprüche erstellt

| Recherchenort | Abschlußdatum der Recherche | Prüfer |
|---|---|---|
| DEN HAAG | 16-06-1988 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument